(19) Europäisches Patentamt
European Patent Office
Office européen des brevets

(11) Veröffentlichungsnummer: **0 223 162**
**A2**

# (12) EUROPÄISCHE PATENTANMELDUNG

(21) Anmeldenummer: **86115439.1**

(51) Int. Cl.[4]: **C07H 17/075** , C12Q 1/34

(22) Anmeldetag: **07.11.86**

(30) Priorität: **19.11.85 DE 3540917**

(43) Veröffentlichungstag der Anmeldung:
**27.05.87 Patentblatt 87/22**

(84) Benannte Vertragsstaaten:
**AT BE CH DE FR GB IT LI NL SE**

(71) Anmelder: **BAYER AG**
**Konzernverwaltung RP Patentabteilung**
**D-5090 Leverkusen 1 Bayerwerk(DE)**

(72) Erfinder: **Koller, Ernst, Dr.**
**Kirchbach 11a**
**A-8082 Kirchbach(AT)**
Erfinder: **Wolfbeis, Otto S., Dr.**
**Im Hoffeld 32**
**A-8046 Graz(AT)**
Erfinder: **Harnisch, Horst, Dr.**
**Heinenbusch 4**
**D-5203 Much(DE)**

(54) **Neue Glykoside, Verfahren zu deren Herstellung sowie Verfahren und Mittel zum Nachweis und zur photometrischen und fluorimetrischen Bestimmung von zuckerspaltenden Enzymen (Glykosidasen).**

(57) Die Erfindung betrifft neue Glykoside, die als Enzymsubstrate unter dem Einfluß von Hydrolasen, speziell von Glykosidasen, ein farbiges und/oder stark fluoreszierendes Anion bilden und zu einer verbesserten photometrischen bzw. fluorimetrischen Bestimmungsmethode für Glykosidasen verwendet werden können.

**EP 0 223 162 A2**

**Neue Glykoside, Verfahren zu deren Herstellung sowie Verfahren und Mittel zum Nachweis und zur photometrischen und fluorimetrischen Bestimmung von zuckerspaltenden Enzymen (Glykosidasen)**

Die Erfindung betrifft neue Glykoside, die als Enzymsubstrate unter dem Einfluß von Hydrolasen, speziell von Glycosidasen, ein farbiges und/oder fluoreszierendes Anion bilden und zu einer verbesserten photometrischen bzw. fluorimetrischen Bestimmungsmethode für Glykosidasen verwendet werden können.

Photometrische Methoden zur Bestimmung von Glykosidasen sind in "Methods of Enzymatic Analysis" (Herausgaber H.U. Bergmeyer, Band IV, Verlag Chemie, 1984) beschrieben worden. Man unterscheidet dabei Methoden der direkten Verfolgung von enzymatischen Reaktionen durch Farbbildung von jenen, wo erst durch Zugabe eines Reagenzes (z.B. Lauge oder nach Azokupplung) Farbstoffe gebildet werden, deren Konzentration dann photometrisch bestimmt wird. Als Substrate werden eingesetzt p-Nitrophenyl-und Naphthylglykoside.

In entsprechender Weise, jedoch mit erhöhter Empfindlichkeit, lassen sich Glykosidasen fluorimetrisch bestimmen. Fluorimetrische Methoden zur Bestimmung von Glykosidasen unter Verwendung von Glykosiden des α-und β-Naphthols, Umbelliferons und 4-Methylumbelliferons sind von Guilbault in "Enzymatic Methods of Analysis" (Pergamon Press, 1973) beschrieben worden. In Analytica Chimica Acta 163, 67 - (1984) wird ein Verfahren zur Bestimmung von β-Galaktosidase mit Hilfe von Resorufin-β-D-Galaktopyranosid auf fluorimetrischem Wege beschrieben. Weiterhin werden in der DOS 34 12 939 einfach oder mehrfach nitrierte Benzpyran -2-one beschrieben die für transmissionsphotometrische Messungen eingesetzt werden können. Viele dieser Substrate sind kommerziell erhältlich.

Mit der vorliegenden Erfindung werden neue Glykoside zur Verfügung gestellt, die als Enzymsubstrate unter dem Einfluß von Hydrolasen, insbesondere von Glykosidasen, zu langwellig absorbierenden und/oder fluoreszierenden Anionen umgesetzt werden.

Die Erfindung betrifft Verbindungen der allgemeinen Formel

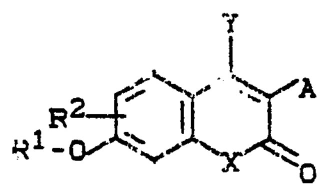

(I)

worin

$R^1$ für einen Zuckerrest

X für -O-oder -NQ-, wobei

Q für Wasserstoff, gegebenenfalls substituiertes $C_1$-$C_4$-Alkyl oder für gegebenenfalls substituiertes $C_1$-$C_4$-Alkoxycarbonyl steht,

Y für Wasserstoff oder Cyano,

A für einen Elektronenakzeptor und

$R^2$ für Wasserstoff oder eine Sulfonsäuregruppe stehen,

mit der Maßgabe, daß A nicht für eine Nitrogruppe stehen kann, wenn $R^2$ und Y für Wasserstoff und X für -O-steht.

Unter Alkyl werden im Rahmen der Anmeldung verzweigte oder geradkettige Alkylreste mit 1 bis 5 C-Atomen, die durch Hydroxy, Halogen, Alkoxy, Alkoxycarbonyl oder Cyano substituiert sein können, verstanden.

Geeignete Zuckerreste $R^1$ in der oben angegebenen allgemeinen Formel (I) sind z.B. die Hexosen α-D-Glukose, β-D-Glukose, α-D-Fruktose, β-D-Fruktose, α-D-Galaktose, β-D-Galaktose, α-und β-D-Glukuronsäure, α-D-und β-D-Mannose, α-D-N-Acetylneuraminsäure, α-und β-D-Acetylglykosamin, β-D-N-Acetylgalaktosamin, α-L-Fukose, α-und β-D-Maltoheptaosid, sowie die Pentosen α-und β-D-Ribose, α-und β-D-Arabinose, α-und β-D-Xylose und α-und β-D-Lyxose.

Bevorzugte Zuckerreste $R^1$ aus der oben genannten Gruppe sind die α-und β-Anomere der Fruktose, D-Glukose, D-Galaktose, D-Mannose und D-Glukuronsäure.

Als Elektronenakzeptoren (Substituent A in der oben angegebenen allgemeinen Formel (I)) sind geeignet Cyano, Nitro, Nitro-, Cyano-oder Sulfophenyl, ein gegebenenfalls substituierter 5 bis 6 gliedriger heteroaromatischer Rest, dem ein gegebenenfalls substituierter Benzo-oder Naphthorest ankondensiert sein kann, wobei als Substituenten geeignet sind Alkyl, Halogen, Alkoxy, $CF_3$, Alkylsulfonyl, Phenyl, Cyclohexyl, Carboxyl, Alkoxycarbonyl, Sulfo, Cyano, Carbamoyl oder Sulfamoyl sowie die N-mono-oder N,N-di-Alkylderivate der beiden letzteren, wobei auch 2 dieser Alkylreste unter Einschluß des gemeinsamen N-Atoms, an das sie gebunden sind, zusammen einen 5-bis 7-gliedrigen Ring bilden, der auch noch ein weiteres Heteroatom enthalten kann. Weiterhin sind als Elektronenakzeptoren geeignet $-SO_2$-R oder -CO-R, wobei R Wasserstoff, Hydroxyl, Alkyl, Alkoxycarbonyl, Cycloalkyl, Alkenyl, Aralkyl, Aryl, Heteroaryl oder auch

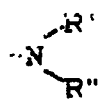

bedeutet, worin R' und R" unabhängig voneinander Wasserstoff, Alkyl, Alkoxycarbonyl oder Aryl sein können. Gut geeignete Beispiele hierfür sind Carboxyl, Sulfo, gegebenenfalls durch $C_1$-$C_4$-Alkyl mono-oder di-substituiertes Carbamoyl oder Sulfamoyl, $C_1$-$C_4$-Alkylsulfonyl, Benzylsulfonyl, Phenylsulfonyl, p-Tolylsulfonyl, p-Chlorphenylsulfonyl, Sulfo-, Nitro-, Cyano-oder Sulfophenyl oder
ein gegebenenfalls durch 1 bis 2 $C_1$-$C_4$-Alkyl, 1 bis 2 Chlor, $C_1$-$C_4$-Alkoxy, Trifluormethyl, $C_1$-$C_4$-Alkylsulfonyl, Cyclohexyl, Phenyl, Carboxyl, $C_1$-$C_4$-Alkoxycarbonyl, Cyano oder Sulfo substituierter Benzoxazol-2-yl-, Benzothiazol-2-yl-, Thiazol-2-yl-, Benzimidazol-2-yl-, Chinazol-4-on-2-yl-, 5-Phenyl-1,3,4-thiadiazol-2-yl, 5-Phenyl-1,3,4-oxadiazol-2-yl, 2-, 3-oder 4-Pyridyl-Rest.

Besonders gut geeignete Elektronenakzeptoren sind gegebenenfalls durch Methyl, Ethyl, Chlor, Methoxy, Ethoxy oder Sulfo substituiertes Benzothiazol-2-yl oder Benzoxazol-2-yl.

Für Q in Verbindungen der allgemeinen Formel (I) sind Methyl, Ethyl und unsubstituiertes $C_1$-$C_4$-Alkoxycarbonyl bevorzugt, wobei R', $R^2$, Y und A die obengenannte Bedeutung zukommt.

Besonders bevorzugte Glykoside sind diejenigen Verbindungen der Formel (I), worin R',$R^2$ und X die oben angegebene Bedeutung haben und
A für einen gegebenenfalls durch Methyl, Ethyl, Chlor, Methoxy, Ethoxy oder Sulfo substituierten Benzothiazol-2-yl oder Benzoxazol-2-yl-Rest und
Y für CN stehen.

Die erfindungsgemäßen Glykoside der Formel (I) mit Y = H können hergestellt werden, indem man Hydroxyverbindungen der Formel

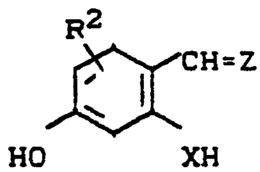

(II)

worin Z für O oder N-Acetyl stehen und X die obengenannte Bedeutung hat, mit einem peracetyliertem Zuckerbromid vorzugsweise in Gegenwart eines anorganischen oder organischen Säurefängers in an sich bekannter Weise umsetzt.

Als Säurefänger eignen sich anorganische und organische Basen, beispielsweise Kalium-, Natrium-oder Silbercarbonat, Calcium-Magnesium-oder Silberoxid, Kalium-und Natriumhydroxid, Quecksilberbromid, Quecksilberacetat, Quecksilbercyanid, Chinolin, Pyridin, Triethylamin, Kollidin, Picolingemische und Dimethylanilin.

Die Reaktion wird vorzugsweise in Gegenwart eines Lösungs-oder Verdünnungsmittels im Temperaturbereich von O bis 150°C durchgeführt.

Als Lösungs-und Verdünnungsmittel eignen sich unter den Reaktionsbedingungen inerte organische Flüssigkeiten wie zum Beispiel Aceton, Benzol, Toluol, Chlorbenzol, Dichlorbenzol, Dichlormethan, Chloroform, Tetrachlormethan, Dichlorethan, Trichlorethylen, Acetonitril, Dioxan, Tetrahydrofuran, Dimethylsulfoxid, Dimethylformamid oder eine der obengenannten organischen Basen wie Chinolin oder Pyridin, vorzugsweise im Gemisch mit Wasser. Man gelangt auf diese Weise bevorzugt zu den β-Glykosiden.

Die entsprechenden α-Glykoside werden in an sich bekannter Weise erhalten durch Schmelzen der Verbindungen (II) mit peracetylierten Zuckern in Gegenwart eines sauren Katalysators wie z.B. $ZnCl_2$ und anschließende fraktionierte Kristallisation aus einem geeigneten Lösungsmittel, um das als Nebenprodukt gebildete β-Anomer abzutrennen. Die Reak tion wird am besten bei Temperaturen zwischen 100 und 150°C durchgeführt. Die fraktionierte Kristallisation erfolgt vorzugsweise aus Ethanol.

Die auf eine der beiden Weisen erhaltenen Zuckerderivate können auf 2 verschiedene Weisen in die gewünschten Verbindungen überführt werden. Man kann sie einerseits zuerst in an sich bekannter Weise - (z.B. in Methanol mit Natriummethylat, Bariummethylat, Kaliummethylat oder Ammoniak oder auch mit wäßrigem Natriumhydroxid, Kaliumhydroxid oder Bariumhydroxid) entacetylieren und anschließend mit einer CH-aciden Verbindung der allgemeinen Formel

$A-CH_2-B$ (III)

worin

A die obengenannte Bedeutung besitzt und

B für Carboxy, $C_1$-$C_4$-Alkoxycarbonyl, Cyano oder Carbamoyl steht, umsetzen.

Die Reaktion wird durch Alkali katalysiert und vorzugsweise in einem Alkohol durchgeführt.

Andererseits kann man die erhaltenen Zuckerderivate zuerst mit einer CH-aciden Verbindung der allgemeinen Struktur (III) in Gegenwart eines sauren oder basischen Katalysators umsetzen und dann erst die Schutzgruppen entfernen.

Eine beträchtliche Verschiebung der Absorptions-bzw. Fluoreszenzmaxima, sowie eine Erniedrigung der $pK_s$-Werte erzielt man durch die Einführung eines elektronenziehenden Substituenten in die 4-Stellung des Cumaringerüsts (Y in der Formel (I)). Dies erfolgt in an sich bekannter Weise durch z.B. oxidative Cyanierung des peracetylierten oder freien Glycosids. Letztere Methode ist empfehlenswerter, da bei der späteren, da basisch katalysierten Entacetylierung die 4-Cyanogruppe angegriffen werden kann.

Neben dem genannten Verfahren zur Herstellung von 3-substituierten Cumarinen sind auch andere Methoden zur Cumarinsynthese geeignet, wie sie z.B. von H. Kröper in Houben-Weyl, Methoden der organischen Chemie, Band 6, Teil 2, Seiten 618 ff beschrieben sind. Erwähnt werden soll auch die Synthese von Glycosiden durch Umsetzung mit entsprechenden 7-Hydroxycumarinen,welche aber in - schlechterer Gesamtausbeute verläuft.

Die neuen Glykoside der Formel (I) sind wasserlöslich, schwach blau (Y = H) oder grünlich (Y = CN) fluoreszierende und zumeist nur wenig farbige Substanzen, die im wäßrigen Medium von Glycosidasen zum stark fluoreszierenden, tief gelben bis roten mesomeren Anion der Formeln

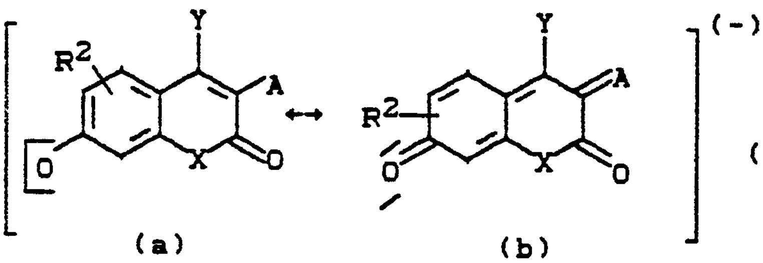

(IV)

gespalten werden.

Die neuen Glykoside der Formel (I) eignen sich daher als Enzymsubstrate zum direkten Glycosidasen-Nachweis in biologischen Materialien. Besonders vorteilhaft können sie zur quantitativen photometrischen oder insbesondere fluorimetrischen Bestimmung von Glycosidasen in der klinischen Analytik verwendet werden, sowohl in homogener Lösung als auch mit Teststreifen.

Durch Wahl eines geeigneten Glykosidrestes läßt sich für jedes Enzym aus der Vielfalt der hydrolytisch wirksamen Enzyme (z.B. Glukosidasen, Galaktosidasen, Glukuronidasen etc.) ein Substrat angeben, welches bevorzugt wird.

Die erfindungsgemäßen Glykoside sind als Enzymsubstrate für Hydrolasen, insbesondere Glykosidasen, sehr gut geeignet.

Wegen der großen Stokes-Verschiebung gegenüber der Absorption der Substrate zeigen sie z.B. keinerlei Überlappung der photometrisch zu verfolgenden Zunahme der Absorption des bei der enzymatischen Hydrolyse entstehenden Anions der Formeln (IV (a) und IV (b)).

Wegen der großen Stokes-Verschiebung wird auch keinerlei störende Überlappung der fluorimetrisch zu verfolgenden, bei der enzymatischen Spaltung von (I) entstehenden, starken Fluoreszenz des Anions - (Formeln IVa und IVb) mit der schwachen Fluoreszenz des eingesetzten Reagenz (I) beobachtet.

Die Verwendung einfachster Filterfluorimeter ist möglich, da die Anregung nicht durch solches UV-Licht erfolgt, bei der sich in der Regel die Untergrundfluoreszenz des biologischen Materials störend bemerkbar macht. Ebenso erübrigt sich die Anwendung aufwendiger Monochromatoren oder Interferenzfilter, wie sie beim Einsatz von Fluorescein-oder Resorufinglycosiden gebraucht werden.

Die Bildung des tief farbigen fluoreszierenden Anions erfolgt überraschenderweise bereits unter schwach sauren Bedingungen (z.B. bei pH 6,0). Hierdurch ist es erstmalig möglich, die Aktivität von Glykosidasen photometrisch oder fluorimetrisch im sichtbaren Spektralbereich in kontinuierlicher Messung im schwach sauren pH-Bereich mit einer so hohen Nachweisempfindlichkeit zu bestimmen, wie es bisher nut mit zeitaufwendigeren nicht-kontinuierlichen Verfahren möglich ist.

Die erfindungsgemäßen Glykoside können als Enzymsubstrate zur Bestimmung von Glycosidase-Aktivitäten in den verschiedensten Körperflüssigkeiten (Serum; cerebrospinale Flüssigkeit; Urin) verwendet werden.

Bei der Bestimmung der Enzymaktivität können die erfindungsgemäßen Verbindungen wegen ihrer meist hinreichenden Wasserlöslichkeit in Form wäßriger Lösungen, gegebenenfalls auf den gewünschten pH-Wert gepuffert, eingesetzt werden. Wenn notwendig, kann durch Zusatz organischer Lösungsmittel (wie z.b. Dioxan, Methylcellosolve, Glykol usw.) die Löslichkeit in Wasser verbessert werden, wobei darauf zu achten ist, daß eine Verminderung der Enzymaktivität nicht eintritt. Eine gute Wasserlöslichkeit ist bei denjenigen Verbindungen gegeben, welche im Molekül eine Sulfogruppe tragen.

Es ist jedoch auch möglich, die neuen Enzymsubstrate in an sich bekannter Weise auf Träger aufzubringen und die Nachweisreaktion in heterogener Phase ablaufen zu lassen, wie dies etwa in Anal. Chem. 55, 498 A (1983) beschrieben ist. Geeignete Trägermaterialien sind z.B. Filterpapier oder auf einem Kunststoff (z.B. Polystyrol) aufgebrachtes mit einem organischen Bindemittel versehenes Siliciumdioxid. Es lassen sich auf diese Weise Teststreifen herstellen, die nach dem Aufbringen der zu untersuchenden Flüssigkeit ihre Farbe ändern bzw. zu fluoreszierenbeginnen und deren Farbe bzw. Fluoreszenz mittels der in der klinischen Analytik üblichen Instrumente gemessen werden kann.

Die quantitative Bestimmung der Enzymaktivität in einer unbekannten Probe ist möglich, wenn man den zeitlichen Verlauf der Änderung der Lichtabsorption bzw. der Fluoreszenzintensität in der wäßrigen Lösung bzw. auf dem Teststreifen mit jenem von Standards mit bekanntem Glykosidasegehalt vergleicht.

Bei den im folgenden beschriebenen Versuchen wurden als Standards folgende Hydrolasenpräparate der Firma Sigma Chemical Co. verwendet:

a) α-Glukosidase (EC 3.2.1.20): Typ V (G 9259) aus Reis.
b) β-Glukosidase (EC 3.2.1.21): Typ I (G 4511) aus Mandeln.
c) α-Galaktosidase (EC 3.2.1.22): (G 6762) aus E coli.
d) β-Galaktosidase (EC 3.2.1.23): (G 1875) aus Rinderleber.
e) β-Glukuronidase (EC 3.2.1.31): (105-2000) aus E.coli.
f) α-Amylase (EC 3.2.1.1): Typ I-A (A 4268) aus Schweinepankreas.
g) β-N-Acetylglukosaminidase (EC 3.2.1.30): (A 4525) aus Rinderniere.

Beispiel 1

1 g (2,2 mMol) 2-Hydroxy-4-(tetra-O-acetyl-β-D-glucopyranosyloxy)benzaldehyd und 0,48 g (2,2 mMol) 2-Benzothiazolylessigester werden in 5 ml absolutem Ethanol unter Erwärmen gelöst. Die warme Lösung wird mit 10 Tropfen Piperidin versetzt und mehrere Stunden bei Raumtemperatur stehengelassen. Dabei scheiden sich aus der Lösung Kristalle der Verbindung der Formel

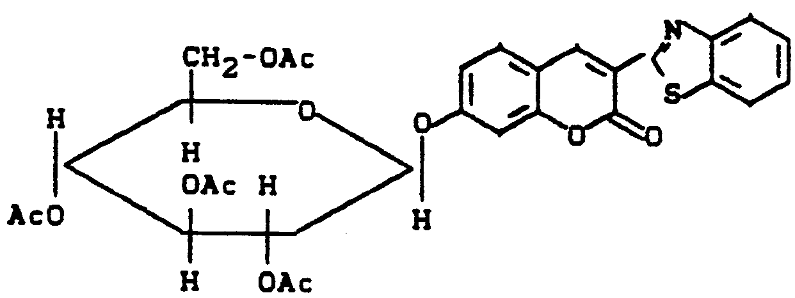

(Ausbeute 0,7 g, entsprechend 52 % der Theorie) ab. Die Reinigung erfolgt durch Umkristallisation aus Eisessig. Man erhält leicht gelbliche Kristalle vom Schmelzpunkt 271-272°C.

Die Verbindung ist löslich, z.B. in Aceton, Dimethylformamid und Dimethylsulfoxid.

In analoger Weise werden unter Verwendung der entsprechenden Ausgangsverbindungen die folgenden Glykoside der allgemeinen Formel

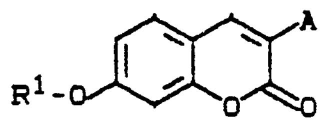

hergestellt.

| Beispiel | $R^1$ | A | Schmelzpunkt |
|---|---|---|---|
| 2 | Tetra-O-acetyl-α-D-glukopyranosyl | 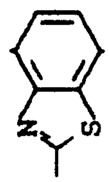 | 265° C |
| 3 | Tetra-O-acetyl-β-D-galaktopyranosyl | 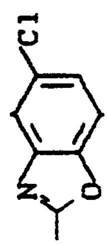 | 281° C |
| 4 | Tetra-O-acetyl-α-D-galaktopyranosyl | 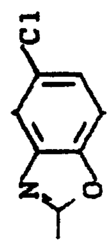 | 273° C |

Beispiel 5

0,62 g (1 mMol) 3-Benzothiazolyl-7-(tetra-O-acetyl-β-D-glucopyranosyloxy)-cumarin werden in 50 ml absolutem Methanol suspendiert, mit etwas festem Natriummethylat versetzt und kurze Zeit unter Rückfluß gekocht. Nach Filtration von Ungelöstem wird die klare Lösung auf ungefähr 20 ml eingeengt und auf 0°C gekühlt. Dabei scheiden sich leicht gelbliche Kristalle der Verbindung der Formel

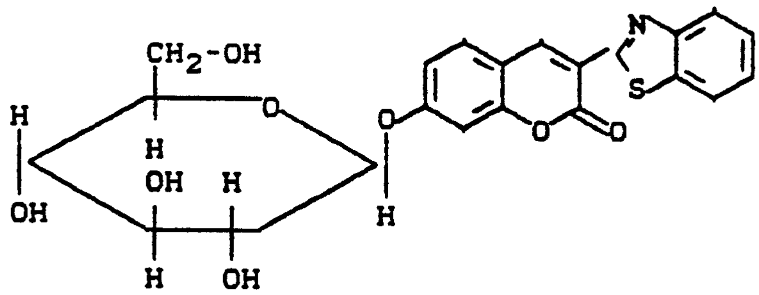

(Ausbeute: 0,28 g, entsprechend 60 % der Theorie) ab. Die Reinigung erfolgt durch Umkristallisation aus einem Gemisch von Ethanol und Wasser. Man erhält leicht gelbliche Kristalle, welche sich bei 238-242°C zersetzen.

Die Verbindung ist löslich, z.B. in Dimethylformamid, Dimethylsulfoxid, Ethylenglykolmonoethylether und z.T. in Wasser.

Zur photometrischen Verfolgung der enzymatischen Hydrolyse mißt man die Extinktion bei 440 nm.

Zur fluorimetrischen Verfolgung der enzymatischen Hydrolyse wählt man Anregungslicht mit einem Maximum bei 440 nm und mißt die Fluoreszenz bei 490 nm. Bei dieser Wellenlänge wird ausschließlich die Fluoreszenz des enzymatisch gespaltenen Substrates erfaßt.

In analoger Weise werden unter Verwendung der entsprechenden Ausgangsverbindungen die folgenden Glycoside der allgemeinen Formel

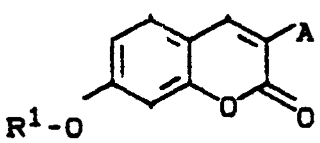

hergestellt.

| Beispiel | $R^1$ | A | Zersetzungspunkt |
|---|---|---|---|
| 6 | α-D-Glukopyranosyl | 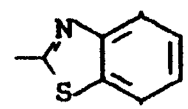 | 235° C |
| 7 | β-D-Galaktopyranosyl | 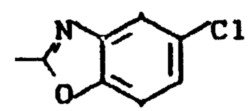 | 273° C |
| 8 | α-D-Galaktopyranosyl | 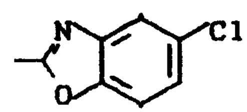 | 256° C |

Beispiel 9

0,93 g (2 mMol) 3-Benzothiazolyl-7-glukopyranosyloxy-cumarin werden in 10 ml Dimethylformamid mit 4 mMol Kaliumcyanid in Form einer 30 %igen wäßrigen Lösung versetzt und die Reaktionsmischung anschließend 1 Stunde bei 40°C gerührt. Nach Kühlung auf 0°C werden 0,1 ml Brom (2 mMol) zugetropft und die Mischung abermals 1 Stunde unter Kühlung gerührt. Dabei scheiden sich orange Kristalle der Verbindung der Formel

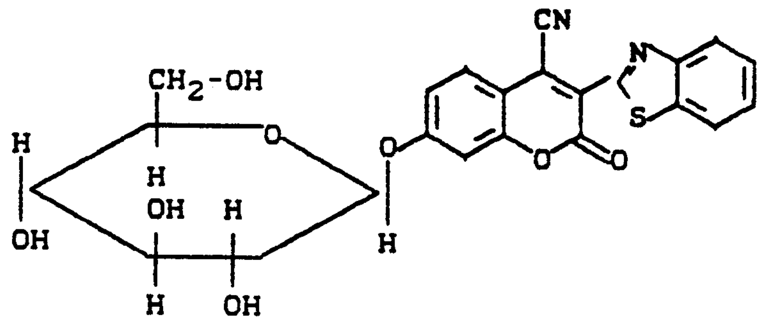

ab.

Die Reinigung erfolgt durch Umkristallisation aus einem Gemisch von Ethanol und Wasser. Man erhält gelb-orange Kristalle, die sich bei 315°C zersetzen.

Die Verbindung ist löslich, z.B. in Dimethylformamid, Dimethylsulfoxid, Ethylenglykolmonoethylether und wenig in Wasser.

Zur photometrischen Verfolgung der enzymatischen Hydrolyse mißt man die Extinktion bei 505 nm. Zur fluorimetrischen Verfolgung der enzymatischen Hydrolyse wählt man Anre gungslicht mit einem Maximum bei 505 nm und mißt die Fluoreszenz bei 595 nm. Bei dieser Wellenlänge wird ausschließlich die Fluoreszenz des enzymatisch gespaltenen Substrates erfaßt.

In analoger Weise werden unter Verwendung der entsprechenden Ausgangsverbindungen die folgenden Glycoside der Formel

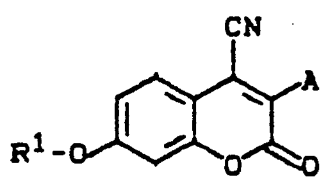

hergestellt:

| Beispiel | $R^1$ | A | Zersetzungspunkt |
|---|---|---|---|
| 10 | α-D-Glukopyranosyl | 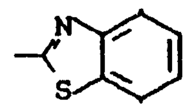 | 311° C |
| 11 | β-D-Galaktopyranosyl | 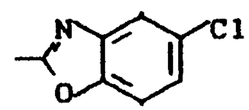 | 305° C |
| 12 | α-D-Galaktopyranosyl | 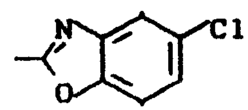 | 301° C |

Beispiel 13

In einem Photometer oder Fluorimeter füllt man bei 25°C die Küvette mit 3 ml einer entsprechenden gepufferten Lösung der Verbindung des Beispiels (5) vom gewünschten pH, stellt die Absorptions-bzw. Anregungswellenlänge auf 440 nm und im Fluorimeter die Emissionswellenlänge auf 490 nm ein, fügt 0,1 ml der auf $\beta$-Glukosidase Aktivität zu bestimmenden Flüssigkeit hinzu und verfolgt die zeitliche Änderung der Absorption bzw. Fluoreszenzintensität über einen Zeitraum von etwa 1 bis 10 Minuten im Vergleich zu vorher angefertigten Eichkurven. Der anfangs lineare Anstieg der Fluroeszenzintensität ist ein direktes Maß für die Enzymaktivität.

## Ansprüche

1. Verbindungen der allgemeinen Formel

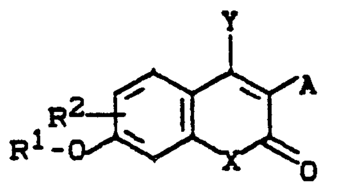

(I)

worin
$R^1$ für einen Zuckerrest,
X für -O-oder -NQ-, wobei
Q für Wasserstoff, gegebenenfalls substituiertes $C_1$-$C_4$-Alkyl oder für ein gegebenenfalls substituiertes $C_1$-$C_4$-Alkoxycarbonyl steht,
Y für Wasserstoff oder Cyano
A für einen Elektronenakzeptor und
$R^2$ für Wasserstoff oder eine Sulfonsäuregruppe stehen,
mit der Maßgabe, daß A nicht für eine Nitrogruppe stehen kann, wenn $R^2$und Y für Wasserstoff und X für -O-steht.

2. Verbindungen der allgemeinen Formel (I) nach Anspruch 1, dadurch gekennzeichnet, daß $R^1$, X, Q, Y und $R^2$ die in Anspruch 1 angegebene Bedeutung haben und
A für Cyano, Nitro, Nitro-, Cyano-oder Sulfophenyl, eine gegebenenfalls substituierten 5-bis 6-gliedrigen heteroatomatischen Rest, dem ein gegebenenfalls substituierter Benzo-oder Naphthorest ankondensiert sein kann, oder für $-SO_2$-R oder -CO-R steht wobei R für Wasserstoff, Hydroxyl, Alkyl, Alkoxycarbonyl, Cycloalkyl, Alkenyl, Aralkyl, Aryl, Heteroaryl oder für

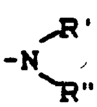

steht, worin R' und R" unabhängig voneinander Wasserstoff, Alkyl, Alkoxycarbonyl oder Aryl sein kann.

3. Verbindungen der allgemeinen Formel (I) nach Anspruch 1, dadurch gekennzeichnet, daß $R^1$, X, Q, Y und $R^2$ die im Anspruch 1 angegebene Bedeutung haben und
A für Carboxyl, Sulfo, $C_1$-$C_4$-Alkoxycarbonyl, für ein gegebenenfalls durch 1 bis 2 $C_1$-$C_4$-Alkylrest substituiertes Carbamoyl oder Sulfamoyl, für $C_1$-$C_4$-Alkylsulfonyl, Benzylsulfonyl, Phenylsulfonyl, p-Tolylsulfonyl, p-Chlorphenylsulfonyl, Nitro-, Cyano-oder Sulfophenyl oder für einen gegebenenfalls durch 1 bis 2 $C_1$-$C_4$-Alkyl, 1 bis 2 Chlor, $C_1$-$C_4$-Alkoxy, Trifluormethyl, $C_1$-$C_4$-Alkylsulfonyl, Cyclohexyl, Phenyl, Carboxyl, $C_1$-$C_4$-Alkoxycarbonyl, Cyano oder Sulfo substituierten Benzoxazol-2-yl-, Benzothiazol-2-yl-, Thiazol-2-yl-, Benzimidazol-2-yl, Chinazol-4-on-2-yl-, 5-Phenyl-1,3,4-thiadiazol-2-yl-, 5-Phenyl-1,3,4-oxadiazol-2-yl-, 2-, 3-oder 4-Pyridyl-Rest steht.

4. Verbindungen der allgemeinen Formel (I) nach Anspruch 1, dadurch gekennzeichnet, daß
$R^1$, X, Q, Y und $R^2$ die im Anspruch 1 angegebene Bedeutung haben und
A für einen gegebenenfalls durch Methyl, Ethyl, Chlor, Methoxy, Ethoxy oder Sulfo substituierten Benzothiazol-2-yl oder Benzoxazol-2-yl-Rest steht.

5. Verbindungen der allgemeinen Formel (I) nach Anspruch 1, dadurch gekennzeichnet, daß $R^1$, X, Q und $R^2$ die im Anspruch 1 angegebene Bedeutung haben, A die in den Ansprüchen 1 bis 4 angegebene Bedeutung hat und Y für CN steht.

6. Verfahren zur Herstellung von Verbindungen der allgemeinen Formel

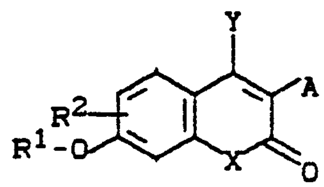

(I)

worin
$R^1$ für einen Zuckerrest,
X für -O-oder -NQ-, wobei
Q für Wasserstoff, gegebenenfalls substituiertes $C_1$-$C_4$-Alkyl oder für ein gegebenenfalls substituiertes $C_1$-$C_4$-Alkoxycarbonyl,
Y für Wasserstoff oder Cyano
A für einen Elektronenakzeptor und
$R^2$ für Wasserstoff oder eine Sulfonsäuregruppe steht,
mit der Maßgabe, daß A nicht für eine Nitrogruppe stehen kann, wenn $R^2$ und Y für Wasserstoff und X for -O-steht,
dadurch gekennzeichnet, daß man Hydroxyverbindungen der Formel

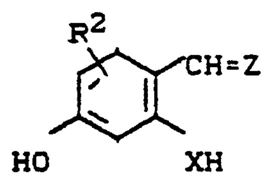

(II)

worin Z für O oder N-Acetyl steht, mit einem peracetyliertem Zuckerbromid umsetzt und das erhaltene Produkt entweder zuerst mit einer $CH_2$ aciden Verbindung der allgemeinen Formel
$A\text{-}CH_2\text{-}B$ (III)
worin
A die obengenannte Bedeutung haben kann und
B für Carboxy, $C_1$-$C_4$-Alkoxycarbonyl, Cyano oder Carbamoyl steht,
umsetzt und anschließend entacetyliert oder zuerst entacetyliert und dann mit der $CH_2$-aciden Verbindung umsetzt und falls Y für CN steht einer oxidativen Cyanierung unterzieht.

7. Verfahren nach Anspruch 6, dadurch gekennzeichnet, daß man die Umsetzung der Hydroxylverbindung der Formel (II) mit einem peracetylierten Zucker in einer Schmelze in Gegenwart eines sauren Katalysators durchführt.

8. Verwendung von Verbindungen der allgemeinen Formel

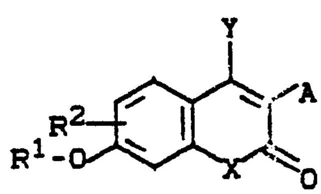

(I)

worin
$R^1$ für einen Zuckerrest,
X für -O-oder -NQ-, wobei
Q für Wasserstoff, gegebenenfalls substituiertes $C_1$-$C_4$-Alkyl oder für ein gegebenenfalls substituiertes $C_1$-$C_4$-Alkoxycarbonyl,
Y für Wasserstoff oder Cyano
A für einen Elektronenakzeptor und

$R^2$ für Wasserstoff oder eine Sulfonsäuregruppe stehen, mit der Maßgabe, daß A nicht fülr eine Nitrogruppe stehen kann, wenn $R^2$ und Y für Wasserstoff und X für -O-steht, als Substrat in einem Testmittel für Glykosidasen.

9. Testmittel für den photometrischen oder fluorometrischen Nachweis von Glykosidasen, dadurch gekennzeichnet, daß sie Verbindungen der allgemeinen Formel

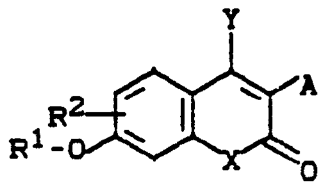

(I)

enthalten, worin
$R^1$ für einen Zuckerrest,
X für -O-oder -NQ-, wobei
Q für Wasserstoff, gegebenenfalls substituiertes $C_1$-$C_4$-Alkyl oder für ein gegebenenfalls substituiertes $C_1$-$C_4$-Alkoxycarbonyl,
Y für Wasserstoff oder Cyano
A für einen Elektronenakzeptor und
$R^2$ für Wasserstoff oder eine Sulfonsäuregruppe steht.
mit der Maßgabe, daß A nicht für eine Nitrogruppe stehen kann, wenn $R^2$ und Y für Wasserstoff und X für -O-steht,

10. Verfahren zur Bestimmung von Glycosidase-Aktivität in einer flüssigen Probe, dadurch gekennzeichnet, daß man die zu untersuchende Probe mit einer Verbindung nach den Ansprüchen 1 bis 5, bzw. einem Testmittel nach Anspruch 9 in Kontakt bringt und die Änderung der Lichtabsorption bzw. der Fluoreszenzintensität in Abhängigkeit von der Zeit mißt.